# EUROPEAN PATENT SPECIFICATION

(11) **EP 4 313 207 B1**
(45) Date of publication and mention of the grant of the patent: **07.05.2025**
(21) Application number: 22720563.0
(22) Date of filing: 30.03.2022
(51) Int. Cl.: A61M 5/00, A61J 1/16

(54) **CARTRIDGE RETAINING TRAY**
KASSETTENRÜCKHALTESCHALE
PLATEAU DE RETENUE DE CARTOUCHE

(30) Priority: 30.03.2021 CA 3113451
(43) Date of publication of application: 07.02.2024
(73) Proprietor: Septodont Holding, 94100 Saint-Maur-Des-Fossés (FR)
(72) Inventor: VIENS, Mathieu, 94100 Saint-Maur-Des-Fossés (FR)
(74) Representative: Icosa
(86) International application number: PCT/EP2022/058428
(87) International publication number: WO 2022/207712

(56) References cited:
- US-A1- 2019 343 721
- US-B2- 10 800 557

## Description

### FIELD OF THE INVENTION

The present invention relates to a system and device for retaining a cartridge which may contain a solution of a medical buffer, such as sodium bicarbonate or other such preparation.

### BACKGROUND OF THE INVENTION

The cartridges referred to in this application generally have a cylindrical configuration and are formed of a suitable glass or plastic material. The cartridges linked to the present invention have needle permeable septum at one extremity and a plunger or piston sealing each cartridge at the other extremity. These cartridges are widely known and used in the industry. It is important that the plunger or piston remains in a static position for the entire period in which the cartridges are present in the system. This is required to maintain stability of the cartridge inside the tray during its storage period.

Cartridge trays are disclosed in US 2019/343721 Al and in US 10 800 557 B2.

### SUMMARY OF THE INVENTION

The invention thus relates to a tray designed to receive at least one cartridge comprising a plunger movable inside the cartridge, said tray comprising: a plurality of recesses at least one recess extending along an engagement axis, each recess being surrounded by a side wall; the or each recess having an upwardly, along the engagement axis, extending post located therein, each recess further comprising a plurality of protruding retaining members extending radially from said side wall into the recess; at least one cartridge placed over one of said posts, wherein the said cartridge tray is configured to retain the cartridge being retained therein inside the recess by said plurality of protruding retaining members, and wherein the post is configured to contact the plunger of the cartridge when the cartridge is inserted in the recess, such that each cartridge being spaced from a bottom wall of said recess.

The cartridge retaining tray of the present invention is designed to retain internal pressure irrespective of the pressure within the system (i.e. inside the cartridge). Generally, the cartridges may be stored/held in a tray with each cartridge being individually retained within the tray. In order to insert a cartridge into the tray, the cartridge is inserted from the top until the plunger or piston of the cartridge contacts the post. Surrounding the post are fins or retaining members which are compressed outwardly by the body of the cartridge and which will then apply a retaining force which radially compresses the cartridge, resulting in a balanced pressure.

A benefit of the present invention is that it allows the plunger to always rest on the post regardless (or within a reasonable range) of its initial position relative to the body of the cartridge when the cartridge is inserted in the tray. The post therefore always abuts the plunger without the bottom of the cartridge touching the bottom of the recess. This results in the constant presence of a resistance enabling the built-up of an internal pressure without any plunger movement.

**In** an embodiment, each retaining members may comprise a retaining tip configured to contact the cartridge. The retaining tip increases stability and safety of the cartridge inside the tray by limiting the contact between the retaining members and the cartridge.

**In** an embodiment, the tray may comprise external slots surrounding the recess for enabling each retaining member to be pushed away from the post. The slots give flexibility to the retaining members which can move radially outward from the post. This configuration allows to have a tray that is easy to make.

**In** an embodiment, the or each recess may comprise a plurality of ribs on the side wall to avoid contact between the cartridge and the side wall of the recess. The ribs thus ensure a proper positioning of the cartridge inside the recess and provide a stop in order to avoid damages of the cartridge against the side wall of the recess.

**In** an embodiment, the post may have a cylindrical, conical, square or cross shape, which is convenient for a cartridge of cylindrical shape.

**In** an embodiment, the tray may have a rectilinear, circular shape.

Another object of the invention is a kit comprising a tray as described above, and one or several cartridges comprising a plunger configured to be stored inside the or each recess of said tray.

The retaining tray may be of any suitable size and indeed, may only hold a single cartridge or up to a substantial number.

### BRIEF DESCRIPTION OF THE DRAWINGS

Having thus generally described the invention, reference will now be made to the accompanying drawings illustrating an embodiment thereof, in which;
Figure 1 is top plan view of a tray for containing a plurality of cartridges;
Figure 2 is a sectional view taken along the lines A-A of Figure 1;
Figure 3 is a side elevational view of the tray;
Figure 4 is a bottom plan view thereof;
Figure 5 is a vertical sectional view of one of the cavities of the tray illustrated in Figure 1;
Figure 6 is a partial horizontal sectional view thereof;
Figure 7 is a perspective view of a horizontal cut through a circular tray, and
Figure 8 is a top plan view of one of the cavities of the tray illustrated in Figure 1.

### DETAILED DESCRIPTION OF THE INVENTION

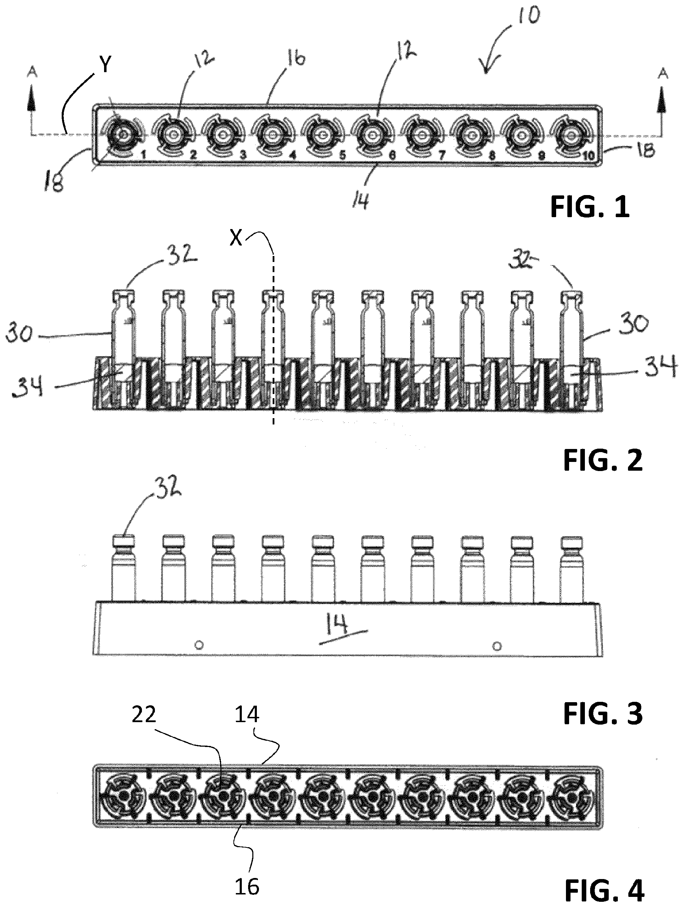

Referring to the drawings in greater detail and by reference characters thereto, there is illustrated in Figures 1 to 4 a tray 10 which presents a number of cartridge holders (or receptacles) generally designated by reference numeral 12. Each cartridge holder 12 extends along an engagement axis X and is designed to cooperate with one cartridge 30. The tray 10 is thus designed to receive a number of cartridges 30.

Tray 10 will include four outer walls 14, 16, 18: a front wall 14 (or deck wall 14), a rear wall 16 and end walls 18. Each cartridge holder 12 extends between the deck wall 14 and the rear wall 16. In a preferred embodiment, the deck wall 14 and the rear wall 16 are parallel to each other and perpendicular to the engagement axis X.

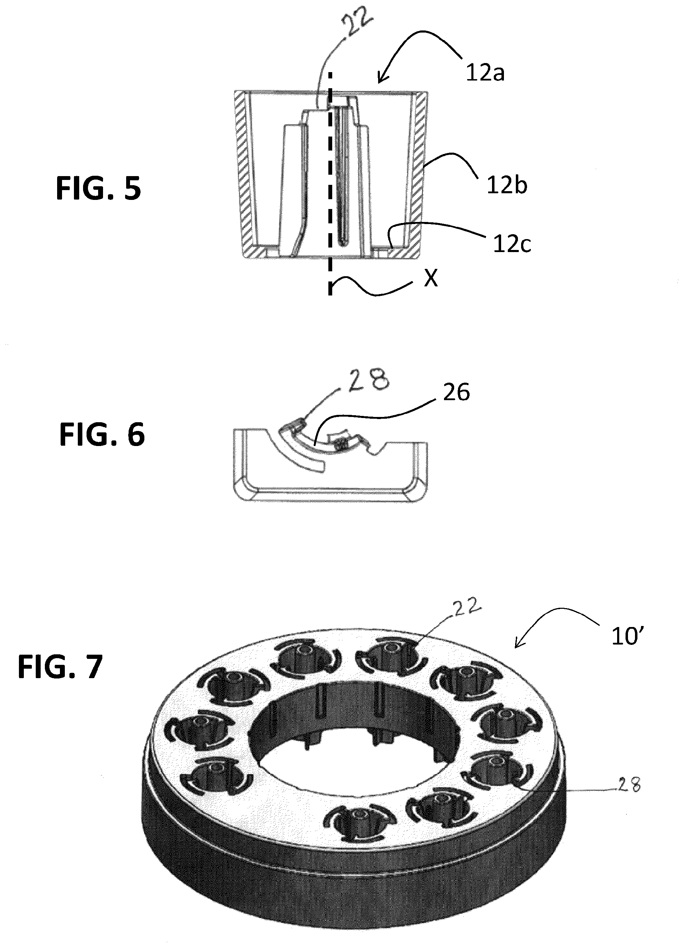

**.** In the illustrated embodiment, there are receptacles 12 for ten cartridges 30. The deck wall 14 and the rear wall 16 extend along a longitudinal axis Y (Figure 1), perpendicular to the engagement axis X of the cartridge holder 12. It will be understood that the tray 10 could be comprised of an unlimited number of receptacles 12 and that the configuration can be other than a longitudinal design. Thus, one could have a circular design, zigzag design, etc. (see for example the circular tray 10' of Figure 7).

The tray 10 may be designed for any size cartridge 30, common sizes including 1mL, 1.8mL and 3mL. Normally, the cartridge 30 would be designed for a 0.8mL fill for a 1 millilitre cartridge. In the depicted embodiments, the cartridges 30 are 0,4mL filled cartridges. Naturally, the tray 10 could be adapted for any desired volume.

Each cartridge holder 12 comprises a recess 12a extending between the deck wall 14 and the rear wall 16. Each recess 12a displays a general cylindrical shape, similar to the shape of a cartridge 30 and presents a size that allows a cartridge 30 to be introduced therein along the engagement axis X. Each recess 12a is surrounded by an internal side wall 12b and comprises a bottom wall 12c.

Each cartridge may be made of any suitable material known, such as glass or rigid plastic material.

Each cartridge holder 12 further comprises, inside the recess 12a, a centrally located cylindrical post 22 extending from the bottom wall 12c along the engagement axis X. The post 22 has a generally cylindrical or slightly conical shape. In some alternative embodiments, the post 22 could present a square shape or a cross shape. A cross shape can reduce amount of material used and function the same way as a conical or cylindrical shape post. The post 22 has a maximum outer diameter such that the cartridge 30 may be inserted onto the post 22 until the post 22 abuts (or contact) the plunger 34 of the cartridge 30.

Cartridges 30 which are designed to be placed in tray 10 each include a pierceable septum 32 (or any type of suitable closing cap) sealing the cartridge at one extremity and a plunger 34 sealing the cartridge at the opposite extremity. The plunger 34 is movable inside the cartridge 34 using for example an external rod pushing onto the plunger 34 to pressurize the liquid in the cartridge in order to eject it at the opposite side (e.g. at the pierced septum 32). The cartridge is placed such that the (in this case) cylindrical post 22 contacts plunger 34. It will be noted that the cartridge wall does not contact the bottom of the cartridge holder 12. Thus, the plunger 34 stays in place and any movements of the plunger 34 is prevented when the cartridge 30 is in the recess 12a, resulting in a better stability of the cartridges 30 in the tray.

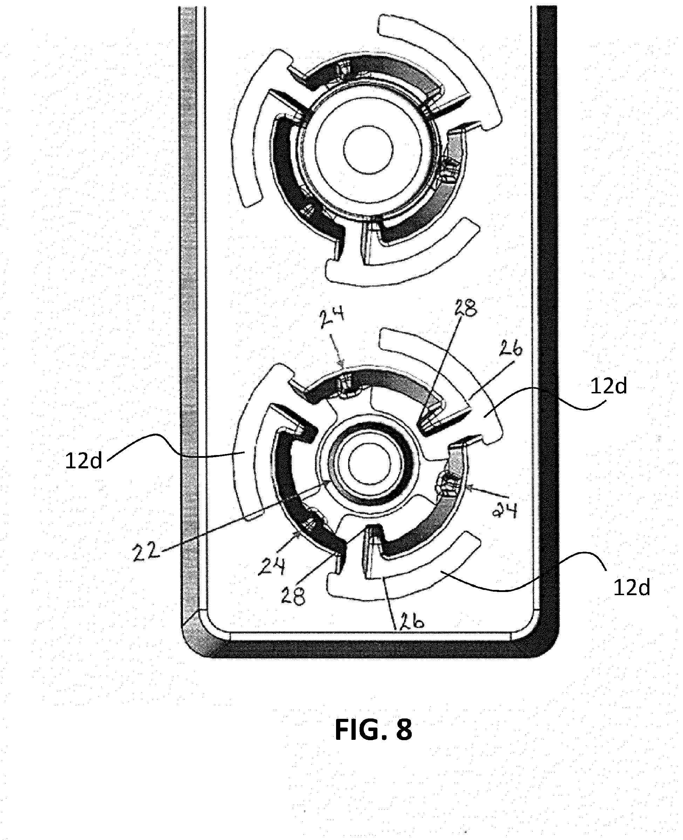

As can be seen on figure 8, the tray 10 comprises a plurality of ribs 24 (in the illustrated embodiment there are three) which are designed to radially support each engaged cartridge 30 (i.e. the outer wall of said cartridge). Each rib 24 thus extends around the post 22, inside the recess 12a, from the bottom wall 12c along the engagement axis X. Each rib 24 preferably protrudes from the inner side wall 12b of the recess 12a towards the post 22. The set of ribs 24 is designed to support the cartridge 30 outer diameter. More precisely, each rib 24 cooperates, by friction, with the outer wall of the inserted cartridge 30 and stabilizes the cartridge 30 on the post 22.

It is important that the cartridge wall does not contact the tray 10 such that constant contact between the cartridge post 22 and plunger 34 is maintained.

Each cartridge holder 12 also includes retaining members (or latches) 26 to radially compress the cartridge 30 in order to generate a friction that retains the cartridge 30 in place even if there is a pressure differential which would tend to move the cartridge plunger. As can be seen on figure 8, each retaining member 26 includes a retaining tip 28. Each retaining member 26 is flexible in order to enable each corresponding retaining tip 28 to move radially closer or further to the post 22 of the recess 12a. In the example illustrated on figure 8, the inner side wall of the recess 12a is partially formed by the retaining member and each retaining tip 18 forms another support rib extending along the engagement axis X. In this embodiment, each cartridge holder 12 further includes external slots 12d surrounding the recess 12a and enabling each retaining member 26 to be pushed away from the post 22. In this embodiment, the diameter of the recess 12a can thus adapt to the inserted cartridge 30 and each tip 28 can cooperate by friction with the outer wall of the inserted cartridge 30.

The tray 10 is thus designed to prevent any movement of the plunger 34 caused by pressure differential - i.e. when the ambient pressure is not equal to the cartridges 30 internal pressure due to fluid expansion, or creation of gas or encountering even a negative ambient pressure.

It will be understood that the above-described embodiment is for purposes of illustration only and that changes and modifications may be made thereto without departing from the scope of the invention.

## Claims

1. A tray (10; 10') designed to receive at least one cartridge (30) comprising a plunger (34) movable inside the cartridge (30), said tray (10; 10') comprising:
at least one recess (12a) extending along an engagement axis (X), each recess (12a) being surrounded by a side wall (12d);
the or each recess (12) having an upwardly, along the engagement axis (X), extending post (22) located therein, each recess (12a) further comprising a plurality of protruding retaining members (26) extending radially from said side wall (12b) into the recess (12a);
wherein the tray (10; 10') is configured to retain the cartridge (30) inside the recess (12) by said plurality of protruding retaining members (26), and wherein the post (22) is configured to abut the plunger (34) of the cartridge when the cartridge is inserted in the recess, such that each cartridge (30) is spaced from a bottom wall (12c) of said recess (12).

2. The tray (10; 10') according to claim 1, wherein each retaining member (26) comprises a retaining tip (28) configured to abut the cartridge (30).

3. The tray (10; 10') according to claim 1 or claim 2, wherein the tray (10; 10') comprises external slots (12d) surrounding the recess (12a) for enabling each retaining member (26) to be pushed away from the post (22).

4. The tray (10; 10') according to any one of claims 1 to 3, wherein the or each recess (12a) comprises a plurality of ribs (24) on the side wall (12b) to avoid contact between the cartridge (30) and the side wall (12b) of the recess (12a).

5. The tray (10; 10') according to any one of claims 1 to 4, wherein the post (22) has a cylindrical, conical, square or cross shape.

6. The tray (10; 10') according to any one of claims 1 to 5, wherein the tray has a rectilinear (10), or circular (10') shape.

7. A kit comprising a tray (10; 10') according to any one of claims 1 to 6, and one or several cartridges (30) comprising a plunger (34) configured to be stored inside the or each recess (12a) of said tray.

## Patentansprüche

1. Eine Schale (10; 10'), die zum Aufnehmen mindestens einer Kartusche (30) ausgebildet ist, umfassend einen in der Kartusche (30) beweglichen Kolben (34), wobei die Schale (10; 10') umfasst:
mindestens eine Vertiefung (12a), die sich entlang einer Einrastachse erstreckt (X), wobei jede Vertiefung (12a) von einer Seitenwand (12d) umgeben ist;
wobei die oder jede Vertiefung (12) einen sich nach oben, entlang der Einrastachse (X), erstreckenden Pfosten (22) aufweist, der darin angeordnet ist, wobei jede Vertiefung (12a) ferner eine Vielzahl von vorstehenden Halteelementen (26) umfasst, die sich radial von der Seitenwand (12b) in die Vertiefung (12a) erstrecken;
wobei die Schale (10; 10') so eingerichtet ist, dass sie die Kartusche (30) durch die Vielzahl vorstehender Halteelemente (26) in der Vertiefung (12) hält, und wobei der Pfosten (22) so eingerichtet ist, dass er den Kolben (34) der Kartusche anschlägt, wenn die Kartusche in die Vertiefung eingesetzt wird, so dass jede Kartusche (30) von einer Bodenwand (12c) der Vertiefung (12) beabstandet ist.

2. Schale (10; 10') nach Anspruch 1, wobei jedes Halteelement (26) eine Haltespitze (28) umfasst, die so eingerichtet ist, dass sie an der Kartusche (30) anschlägt.

3. Die Schale (10; 10') nach Anspruch 1 oder 2, wobei die Schale (10; 10') äußere Schlitze (12d) umfasst, die die Vertiefung (12a) umgeben, damit jedes Halteelement (26) vom Pfosten (22) weggeschoben werden kann.

4. Schale (10; 10') nach einem der Ansprüche 1 bis 3, wobei die oder jede Vertiefung (12a) mehrere Rippen (24) an der Seitenwand (12b) umfasst, um einen Kontakt zwischen der Kartusche (30) und der Seitenwand (12b) der Vertiefung (12a) zu vermeiden.

5. Schale (10; 10') nach einem der Ansprüche 1 bis 4, wobei der Pfosten (22) eine zylindrische, konische, quadratische oder kreuzförmige Form aufweist.

6. Schale (10; 10') nach einem der Ansprüche 1 bis 5, wobei die Schale eine gerade (10) oder kreisförmige (10') Form aufweist.

7. Satz, der eine Schale (10; 10') nach einem der Ansprüche 1 bis 6 und eine oder mehrere Kartuschen (30) umfasst, die einen Kolben (34) umfassen, der so eingerichtet ist, dass er in der oder jeder Vertiefung (12a) der Schale gelagert wird.

## Revendications

1. Plateau (10 ; 10') conçu pour recevoir au moins une cartouche (30) comprenant un piston (34) mobile à l'intérieur de la cartouche (30), ledit plateau (10 ; 10') comprenant :
au moins un évidement (12a) s'étendant le long d'un axe d'engagement (X), chaque évidement (12a) étant entouré par une paroi latérale (12d) ;
le ou chaque évidement (12) comportant un montant (22) s'étendant vers le haut, le long de l'axe d'engagement (X), situé à l'intérieur de celui-ci, chaque évidement (12a) comprenant en outre une pluralité d'éléments de retenue saillants (26) s'étendant radialement depuis ladite paroi latérale (12b) dans l'évidement (12a) ;
dans lequel le plateau (10 ; 10') est configuré pour retenir la cartouche (30) à l'intérieur de l'évidement (12) par ladite pluralité d'éléments de retenue en saillie (26), et dans lequel le montant (22) est configuré pour venir en butée contre le piston (34) de la cartouche lorsque la cartouche est insérée dans l'évidement, de telle sorte que chaque cartouche (30) soit espacée d'une paroi inférieure (12c) dudit évidement (12).

2. Plateau (10 ; 10') selon la revendication 1, dans lequel chaque élément de retenue (26) comprend une pointe de retenue (28) configurée pour venir en butée contre la cartouche (30).

3. Plateau (10 ; 10') selon la revendication 1 ou la revendication 2, dans lequel le plateau (10 ; 10') comprend des fentes externes (12d) entourant l'évidement (12a) pour permettre à chaque élément de retenue (26) d'être poussé à l'écart du montant (22).

4. Plateau (10 ; 10') selon l'une quelconque des revendications 1 à 3, dans lequel le ou chaque évidement (12a) comprend une pluralité de nervures (24) sur la paroi latérale (12b) pour éviter le contact entre la cartouche (30) et la paroi latérale (12b) de l'évidement (12a).

5. Plateau (10 ; 10') selon l'une quelconque des revendications 1 à 4, dans lequel le montant (22) a une forme cylindrique, conique, carrée ou en croix.

6. Plateau (10 ; 10') selon l'une quelconque des revendications 1 à 5, dans lequel le plateau a une forme rectiligne (10), ou circulaire (10').

7. Kit comprenant un plateau (10 ; 10') selon l'une quelconque des revendications 1 à 6, et une ou plusieurs cartouches (30) comprenant un piston (34) configuré pour être stocké à l'intérieur du ou de chaque évidement (12a) dudit plateau.
